# EUROPÄISCHE PATENTANMELDUNG

(11) **EP 1 985 270 A2**
(43) Veröffentlichungstag der Anmeldung: **29.10.2008**
(21) Anmeldenummer: 08154977.6
(22) Anmeldetag: 23.04.2008
(51) Int. Cl.: A61F 13/02

(54) **Expandierbarer Wundverband**

(30) Priorität: 24.04.2007 DE 102007019622
(71) Anmelder: Riesinger, Birgit, 48346 Ostbevern (DE)
(72) Erfinder: Riesinger, Birgit, 48346 Ostbevern (DE)
(74) Vertreter: Michalski Hüttermann & Partner Patentanwälte

(57) **Zusammenfassung**

Gegenstand der Erfindung ist ein Wundverband (100; 200; 300; 400), insbesondere für stark exsudierende Wunden, aufweisend (i) eine Flüssigkeiten absorbierende Einheit (10) und (ii) ein folienartiges, an der gesunden Haut um die Wunde herum zu befestigendes Abdeckungselement (3; 13), wobei die Flüssigkeiten absorbierende Einheit bei Flüssigkeitsaufnahme an Volumen gewinnt und in diesem Zustand den jeweiligen Raum zwischen der Wundoberfläche und dem Abdeckungselement (3; 13) wenigstens teilweise ausfüllt. Der Wundverband ist, dadurch gekennzeichnet, dass die Flüssigkeiten absorbierende Einheit durch das Abdeckungselement (3; 13) abgedeckt ist, und das Abdeckungselement (3; 13) Mittel aufweist, die dazu beitragen, dass das Abdeckungselement in seinem auf der Haut des Patienten befestigten Zustand der Volumenzunahme der Flüssigkeiten absorbierenden Einheit nachgibt und sich kontinuerlich ausdehnt (Fig. 6).

## Beschreibung

Die Erfindung betrifft einen Wundverband, insbesondere für stark exsudierende Wunden, aufweisend eine Flüssigkeiten absorbierende Einheit und ein folienartiges, an der gesunden Haut um die Wunde herum zu befestigendes Abdeckungselement, wobei die Flüssigkeiten absorbierende Einheit bei Flüssigkeitsaufnahme an Volumen gewinnt und in diesem Zustand den jeweiligen Raum zwischen der Wundoberfläche und dem Abdeckungselement wenigstens teilweise ausfüllt.

Der Begriff "Flüssigkeiten absorbierende Einheit" bezeichnet eine Einheit, die auf die Wunde aufbringbar ist und Flüssigkeiten, insbesondere Exsudate aufnimmt. In der vorliegenden Erfindung wird dieser Begriff Synomym mit dem Begriff "Wundauflage" verwendet.

Zur Klärung des im folgenden verwendeten Begriffes "Abdeckungselement" wird darauf hingewiesen, dass dieses Abdeckungselement keine Wundauflage im Sinne der Wundtherapie bezeichnet, da es keinerlei Kontakt mit der Wundoberfläche haben soll. Das Abdeckungselement soll lediglich das auf die Wunde aufgelegte, mit superabsorbierender Substanz versehene Verbandmaterial an dem Körper des Patienten fixieren.

Die zur Abdeckung einer Wundauflage verwendeten Folien, wie eine an sich bekannte, unter der Marke OpSite vertriebene Inzisionsfolie, ist eine elastische, wasserdichte Polyurethan-Folie, die im Einsatz bis zum Wundrand reicht, den Wundbereich dicht abdeckt und das Wandern pathogener Hautkeime des Patienten in die Wunde verhindert. Inzisionsfolien sind jedoch klebebeschichtet und damit ganzflächig und fest mit dem Verbandmaterial zu verbinden. Die Inzisionsfolie kann das Quellvermögen einer Flüssigkeiten absorbierdneden Einheit, wie z.B. eines mit superabsorbierender Substanz (SAP) durchsetzten Verbandmaterials, trotz ihrer Elastizität einschränken, da die Folie über relativ starke Rückstellkräfte verfügt, die das Entfalten des maximalen Quellvermögens der Wundauflage verhindern können.

Aus DE 100 58 439 der Anmelderin ist ein wasserdichter Folienabschnitt bekannt, mit dem eine in einer flüssigkeitsdurchlässigen Hülle befindliche und in einer Matte verteilte superabsorbierende Substanz abgedeckt und am Körper des Patienten fixiert werden kann. Allerdings ist die erforderliche Elastizität des zu abdeckenden Folienmaterials nicht berücksichtigt worden, zumal aus dem Grund, dass lediglich die Elastizität der Hülle von Relevanz schien.

Es stellt sich demnach die Aufgabe, einen Wundverband der eingangs genannten Art zu konzipieren, der sein Quellvermögen ungehindert entfalten kann.

Erfindungsgemäß ist diese Aufgabe dadurch gelöst, dass
- die Flüssigkeiten absorbierende Einheit durch das Abdeckungselement abgedeckt ist, und
- das Abdeckungselement Mittel aufweist, die dazu beitragen, dass das Abdeckungselement in seinem auf der Haut des Patienten befestigten Zustand der Volumenzunahme der Flüssigkeiten absorbierenden Einheit nachgibt und sich kontinuerlich ausdehnt.

Das Ausdehnen bzw. Nachgeben erfolgt bis zur Erreichung des maximalen bzw. eingestellten Quellvermögens der Flüssigkeiten absorbierenden Einheit (Wundauflage). Auf diese Weise ist sichergestellt, dass die Flüssigkeiten absorbierende Einheit ungehindert Exsudate aus der Wunde aufnehmen und so zur Förderung der Wundheilung beitragen kann.

Bevorzugt ist dabei vorgesehen, dass es sich bei der Flüssigkeiten absorbierenden Einheit um eine Einheit enthaltend superabsorbierende Substanz handelt, die zwischen der jeweiligen Wundoberfläche und dem Abdeckungselement angeordnet ist.

Superabsorbierende Substanzen weisen ein extrem hohes Bindungsvermögen für Flüssigkeiten auf. So können bestimmte, als Superabsorber bezeichnete Polyacrylate bis zu dem 1000-fachen ihres Eigengewichts an Wasser binden. Sie eignen sich daher für die Behandlung von stark exsudierenden, odematösen Wunden, bei denen eine große Menge Exsudat aus der Wunde abgeführt werden muß.

Solche Superabsorber enthaltenden Flüssigkeiten absorbierenden Einheiten bzw. Wundauflagen sind z.B. aus den Offenlegungsschriften DE 100 58 439 und EP 15 07498 der Anmelderin bekannt, auf deren Inhalt hier vollumfänglich Bezug genommen wird. In diesen Schriften sind Wundauflagen offenbart, die aus einer Matte enthaltend Superabsorber sowie einer Hülle bestehen.

Gleichwohl kann die Superabsorber enthaltende Flüssigkeiten absorbierende Einheit z.B. auch aus einer Schüttung oder einem Pressling aus Superabsorberpartikeln bestehen. Auch eine solche Einheit fällt unter die vorliegende Definition der Wundauflage.

Die Wasseraufnahme durch besagte Einheit geht mit einer entsprechenden Volumenzunahme einher. Aus diesem Grunde ist bei Verwendung von Superabsorbern die erfindungsgemäße Wundabdeckung besonders vorteilhaft.

Die Befestigung des Abdeckungselements auf der gesunden Haut um die Wunde herum erfolgt bevorzugt mit Hilfe eines medizinischen Klebemittels, wie es z.B. für Pflaster verwendet wird; insbesondere mit Hilfe eines nicht allergenen Klebemittels, oder eines handelsüblichen Pflasters. Bevorzugt ist dabei vorgesehen, dass das Abdeckungselement eine kontinuierliche Klebefläche in seinem Randbereich aufweist. Hier kann z.B. ein Klebestreifen vorgesehen sein, der vor Benutzung mit einem Abdeckstreifen kaschiert ist.

Auf diese Weise wird erreicht, dass das Abdeckungselement auf die Wundauflage bzw. auf die superabsorbierende Substanz (SAP) und damit auf die Wundoberfläche keine nennenswerte Gegenkräfte ausübt, obwohl die Flüssigkeiten absorbierende Einheit, z. B. die Wundauflage, auf Grund der Kapillarwirkung bzw. osmotischer Kräfte stark aufquellen und ihr Volumen beachtlich vergrößern kann. Es sei darauf hingewiesen, dass es sich bei den vorliegend diskutierten Wunden um stark sezernierende Wunden, wie Ulcus cruris, handelt, bei deren Heilung eine möglichst große Saugkapazität der Wundauflage vorteilhaft sein kann.

Das erfindungsgemäße Abdeckungselement kann aus einer streckbaren, plastisch und/oder elastisch verformbaren Folie gefertigt sein. Insbesondere ist hier eine plastische Verformbarkeit bevorzugt, da in dieser Ausführungsform die Rückstellkräfte besonders gering sind.

Das das Abdeckungselement kann jedoch ebenfalls aus einem streckbaren, plastisch und/oder elastisch verformbaren Gewebe oder Gewirke gefertigt sein.

Ebenso kann alternativ oder zusätzlich hierzu vorgesehen sein, dass das Abdeckungselement wenigstens teilweise gefaltet ist.
In diesem Fall entfaltet sich das Abdeckungselement bei Volumenzunahme der Wundauflage balgenartig, ohne der Wundauflage nennenswerte Rückstellkräfte entgegenzusetzen. In dieser Ausführungsform kann das Abdeckungselement aus einer verhältnismäßig steiferen Folie hergestellt sein. Vorgesehen kann dabei z.B. sein, dass in einem Folienabschnitt zwei sich gegenüber liegende Falten eingearbeitet sind. Hierzu wird auf die Abbildungen verwiesen.

Unter dem Begriff "Folie" bzw. "folienartiges Element" soll im Folgenden ein flächiges und flexibles Element verstanden werden, das sich für die Abdeckung im obigen Sinne eignet. Nach der diesseitigen Definition können z.B. auch bestimmte Netze unter den Begriff "Folie" fallen. Bevorzugt ist jedoch an Folien im engeren und eigentlichen Sinne gedacht.

Insbesondere kann es sich bei der Folie zumindest abschnittsweise um eine metallische oder metallbedampfte Folie handeln, wie sie auch als Wärmeschutz-, Erste Hilfe- oder Notfallfolie bezeichnet bzw. verwendet wird. Die Verwendung einer solchen Folie kann dazu beitragen, die Temperatur im Wundbereich zu erhöhen, da sie die Körperwärme reflektiert, und so den Heilungsprozess fördert.

Die Folie kann ferner ebenso aktiv wärmende Bestandteile aufweisen, die z.B. durch Ausübung von Druck oder ähnliches aktiviert werden.

So können z.B. kleine Pads vorgesehen sein, die aus einer Mischung von Eisenpulver, Salz, Aktivkohle und Wasser bestehen. Nach der Aktivierung gelangt Sauerstoff in diese Pads und reagiert mit dem Eisenpulver; dabei wird Wärme frei.

Ebenso können kleine Kissen vorgesehen sein, die eine Salzlösung (z.B. Natriumazetat) enthalten, die bei Aktivierung unter Freisetzung von Wärme kristallisiert.

Die genannten aktiv wärmenden Bestandteile können auch an einer der Abdeckfolie unterlegten Folie angeordnet sein.

Die Folie kann weiterhin zumindest abschnittsweise lichtdurchlässig und/oder durchsichtig gestaltet sein. Vorteil dieser Ausgestaltung ist einerseits, dass so eine bessere visuelle Kontrolle durch den Patienten und/oder das behandelnde Personal ermöglicht wird; andererseits kann die Abdeckung so bei Gabe einer Lichttherapie an Ort und Stelle verbleiben. Lichttherapien sind z.B. bei Neurodermitis angezeigt. Dabei wird insbesondere mit ultraviolettem Licht (insbesondere UV-B) gearbeitet. Andere Indikationen verlangen z.B. eine Behandlung mit infrarotem Licht. Entsprechend kann vorgesehen sein, dass die verwendete lichtdurchlässige Folie in ihren Transmissionseigenschaften an die spektralen Eigenschaften des für die Therapie verwendeten Lichts angepasst ist. So würde man beispielsweise bei Neurodermitistherapie eine UV-durchlässige Folie verwenden.

Die Folie kann überdies aus einem semipermeablen Material bestehen, das z.B. für wasserdampfdurchlässig ist, nicht jedoch durchlässig für Flüssigkeiten. Solche Materialien sind z.B. unter dem Begriff Goretex (PTFE) bekannt.

Bevorzugt ist überdies vorgesehen, dass die Folie wärmeresistent, unterdruckresistent und/oder UV-resistent ist.

Die Folie kann Perforationen aufweisen, die entweder für eine Erhöhung der Wasserdampfpermeabilität dienen oder die Folie, wenn sie z.B. auf eine Rolle angeordnet ist, leichter abreissbar machen.

Die Folie kann ferner ein öffenbares Fenster aufweisen, durch welches z.B. die darunter liegende Flüssigkeiten absorbierende Einheit leicht entnommen erden kann, während die Folie auf der Haut verbleibt. Das öffenbare Fenster kann z.B. wiederverschließbar ausgestaltet sein, so z.B. mit Hilfe eines Klettverschlusses.

Die Folie kann bevorzugt künstliche Spinnenseiden-Folie bzw. ein Spinnenseiden-Gewebe aufweisen sein. Hierbei handelt es sich um ein solches Material, dessen Fäden eine Dehnung und Elastizität aufweisen, die des bekannten Materials KEVLAR nach Angaben der Patentschrift EP 0 707 645 B1 überschreiten können.

Die Verwendungsmöglichkeit einer künstlichen Spinnenseidenfolie im Zusammenhang mit Wundauflagen ist zwar in der für die US-Schrift 7,057,023 bereits offenbart. Allerdings ist hier von dem Begriff "wound dressing" die Rede, es handelt sich also um die Verwendung als Wundauflage mit unmittelbarem Kontakt zur Wunde. Hierdurch ist die erfindungsgemäße Verwendung als Abdeckungselement zur Abdeckung der eigentlichen Wundauflage nicht vorweggenommen. Bei der erfindungsgemäßen Verwendung kommt die Spinnenseidenfolie nicht mit der Wunde in Berührung; überdies kommen hier andere Effekte zum Tragen als bei der in der US-Schrift 7,057,023 offenbarten Verwendung in direktem Wundkontakt.

Ferner kann das Abdeckungselement aus natürlichem Seidengewebe gefertigt sein, dessen Fäden aus dem Kokon des Götterbaumspinners *Samia cynthia ricini (= Philosamia ricini)* des Schmetterlings der Familie Saturniidae besteht. Diese natürlichen, als "Eri-Seide" in Indien bekannte Seidenfäden werden zur Herstellung von Feintextilien verwendet.

Das Abdeckungselement kann außerdem auch aus modifizierter Chitosanfolie hergestellt sein. Als modifizierte Chitosanfolie kommt eine solche in Betracht, die mit die elastischen Eigenschaften verbessernden, an sich bekannten Zusätzen versehen ist.

Es ist möglich, aus vorgenannten Materialien ebenfalls die die superabsorbierende Substanz (SAP) umgebenden Hüllenwände, die sich im eingesetzten Zustand unterhalb des Abdeckungselementes befinden, herzustellen.

Weiterhin ist möglich, das Abdeckungselement aus herkömmlichen Kunststofffolien, beispielsweise aus Polyurethan- oder Polyolefinfolie auf der Weise zu formen, dass sie trotz unzureichender eigener Elastizität die vorgenannte Aufgabe erfüllen können. So beispielsweise kann das vorgefertigte Abdeckungselement wenigstens teilweise gefaltet sein.

Dabei kann die superabsorbierende Substanz in einem verhältnismäßig weniger saugfähigen Material, wie Cellulose-Vlies oder dessen Derivaten, verteilt sein.

Die durch das Abdeckungselement gemäß Erfindung abzudeckenden Wundauflagen können aktive, interaktive bzw. bioaktive Wundauflagen sein. Dementsprechend können der die superabsorbierende Substanz aufweisenden, cellulosenartigen Vliesmatte Hydrokolloid-, Hydrogel-, Alginat- oder Hydrofasern, sowie Hyaluronsäureesterderivate, Aktivkohle, Madenspeichel (getrocknet oder Recombinant), Wachstumsfaktoren etc. beigemischt sein. Beigemengt werden können auch Kurzfasern aus künstlicher Spinnenseide. Zwar können die genannten Beimischungen die Saugfähigkeit der Matte beeinträchtigen, jedoch kann der Anteil an superabsorbierender Substanz entsprechend vergrößert werden.

Die die superabsorbierende Substanz aufweisende Flüssigkeiten absorbierende Einheit kann von wenigstens einer weiteren Matte unterlegt und von einer äußeren Umhüllung umgeben sein. Die Matte kann gelocht sein. Als Materialien für die weitere Matte kommen Schaumstoffe, Vlies oder Gewebe in Frage. Die Matte kann u. a. aus Seidenschaum gefertigt sein, der beispielsweise aus einem Seidenmaterial besteht, das dem der künstlichen Spinnenseide identisch oder ähnlich ist.

Weitere Einzelheiten der Erfindung sind nachfolgenden Ausführungsbeispielen zu entnehmen. Die Figuren zeigen:
- Figuren 1a, 1b: einen Wundverband, bestehend aus einer mit superabsorbierender Substanz angereicherten Vliesmatte und einem Sachet, in einer schematischen Seitenansicht vor und nach Absorption;
- Figuren 2a, 2b: einen zweiten Wundverband, bestehend aus einem Sachet gemäß Fig. 1, unterlegt mit einer gelochten, schwammartigen Matte, mit einer äußeren Umhüllung, ebenfalls in einer schematischen Seitenansicht vor und nach Absorption;
- Figuren 3a, 3b: einen anderen Wundverband, bestehend aus einem flüssigkeitsdurchlässigen Säckchen und darin enthaltener granulatartiger superabsorbierender Substanz, ebenfalls in einer schematischen Seitenansicht vor und nach Absorption;
- Fig. 4: ein gefaltetes Abdeckungselement in einer perspektivischen Ansicht;
- Fig. 5: einen Schnitt A-A gemäß Fig. 4;
- Fig. 6: das auf die Haut des Patienten angeklebte, entfaltete Abdeckungselement gemäß Fig. 4.

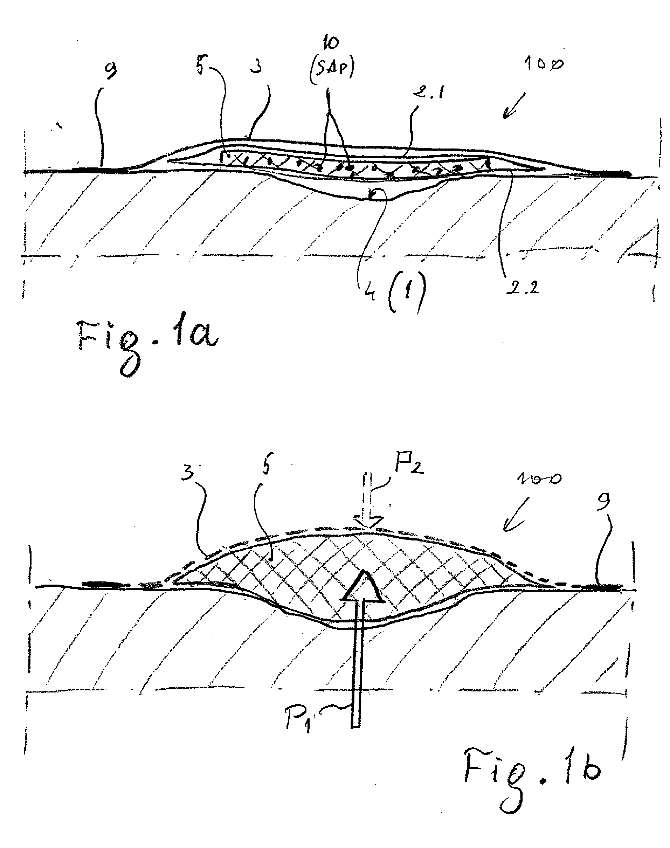

In Fig. 1a ist ein Wundverband 100 dargestellt, aufweisend eine Hülle 2 in Form eines flachen Sachet mit einer darin untergebrachten, vliesartigen Matte 5. Die Hülle 2 besteht aus zwei zueinander kongruenten, flüssigkeitsdurchlässigen, im vorliegenden Fall rechteckigen Hüllenwänden 2.1, 2.2, welche miteinander über eine umlaufende Ultraschallnaht (nicht dargestellt) verbunden sind.

Die Hülle 2 besteht aus einem flüssigkeitsdurchlässigen, schleimhautverträglichen Kunststoff, an dem die Wundsekrete nicht oder kaum haften. Dies ermöglicht den Transport von flüssigen Wundsekreten in die Matte 5. Die Wundsekrete treten durch die Hülle 2 hindurch und werden durch die mit dem Superabsorber angereicherte Matte 5 aufgesaugt.

Die Matte 5 kann ein- oder mehrlagig sein. Es wurde eine etwa 2mm dicke Matte 5 von Typ Airlaid gewählt, welche aus einem Vlieskern 7 und zwei mit dem Vlieskern flächig verbundenen äußeren Schichten 17.1, 17.2 aus 0,10 mm dicker, ebenfalls vliesartigen Folie besteht. Das Vliesmaterial enthält Cellulose und deren Derivate. Die flächige Verbindung der äußeren Schichten 17.1, 17.2 mit dem Vlieskern 7 wurde erreicht in einem Spalt zweier erhitzter, sich gegenläufig drehenden Prägewalzen (nicht dargestellt). Dabei erzeugten die Vorsprünge an den Prägewalzen ein aus längs und quer orientierten Perforationsreihen bestehendes Muster, bei dem die Perforationen an den äußeren Schichten 17.1, 17.2 jeweils rund sind.

In der Matte 5 sind Feststoffpartikeln einer superabsorbierenden Substanz 10, im Weiteren Superabsorber genannt, gleichmäßig verteilt, wobei der Gewichtsanteil an Superabsorber in der Matte 5 etwa 25% beträgt. Der Superabsorber ist aus der Gruppe vernetzte Polyacrylsäure, Hersteller: Stockhausen GmbH, ausgewählt worden.

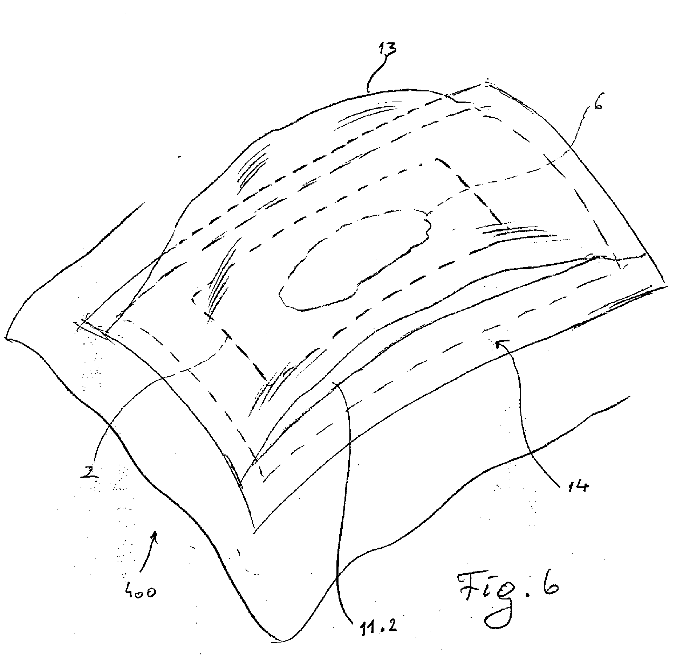

Wie in Fig. 1a gezeigt, ist das sich im trockenem Zustand befindliche Sachet 8 auf eine Wunde 1 derart flach gelegt, dass die im Sachet liegende Matte 5 mit ihrem Rand auch die gesunden Hautareale außerhalb einer Wundkontur 6 (vgl. Fig. 6) abdeckt. Danach wurde auf das Sachet 8 ein hoch elastisches, transparentes Abdeckungselement 3 gelegt, dessen umlaufender Rand 9 an seiner wundzugewandten Seite klebebeschichtet (Klebefläche 14) ist. Unmittelbar vor dem Einsatz wurde ein nicht dargestelltes Abziehstreifen von der Klebefläche 14 entfernt.

Das Abdeckungselement 3 besteht aus künstlicher Spinnenseidenfolie, die sich durch hohe Elastizität und Belastbarkeit auszeichnet. Die Festigkeit der natürlichen Spinnenseiden-Fasern überschreitet die des Stahls (7,8 vs. 3,4 g/Denier), Auch die Elastizität der natürlichen Spinnenseiden-Fasern ist groß und überschreitet beispielsweise die des unter Handelsnamen KEVLAR bekannten Gewebematerials. Es wird jedoch angenommen, dass die wirklichen mechanischen Eigenschaften der künstlichen Spinnenseidenfolie gegenüber der natürlichen höchstens 90% erreichen können.

Außerdem ist ein unerwünschter Effekt der Spinnenseide festzustellen, nämlich die Abschwächung der Elastizität derselben bei Einwirkung von Feuchtigkeit. Das erfindungsgemäße, aus Spinnenseidenfolie gefertigte Abdeckungselement 3 hat dahingegen keinen unmittelbaren Kontakt mit der Feuchtigkeit, da die äußere Oberfläche der nach außen weisenden Hüllenwand 2.1 trotz Feuchtigkeitsaufnahme durch die Matte 5 trocken bleibt. Aus dem Grund wird angenommen, dass die Spinnenseidenfolie, insbesondere die künstliche, keine wesentlichen Verluste an Elastizität aufweist.

Dabei soll angemerkt sein, dass auch die Hüllenwand (2.1, 2.2) wenigstens teilweise aus künstlicher Spinnenseiden-Folie oder künstlichem Spinnenseiden-Gewebe bzw. aus natürlichem Seidengewebe bestehen kann.

Die Kapillarwirkung, die osmotischen Kräfte sowie die Saugkraft des Superabsorbers bewirken eine starke Volumenzunahme der Matte 5 beim Aufsaugen von Wundflüssigkeiten. Die wundzugewandte Hüllenwand 2.2 neigt zum Kontakt mit einer Wundoberfläche 4. Die Summe der genannten Kräfte zeigt schematisch die Andruckkraft P1 (Pfeile Fig. 1b). Da das Abdeckungselement 3 sehr elastisch ist, übt es keine nennenswerten Gegenkräfte (Andruckkraft P2) auf die Wundauflage aus.

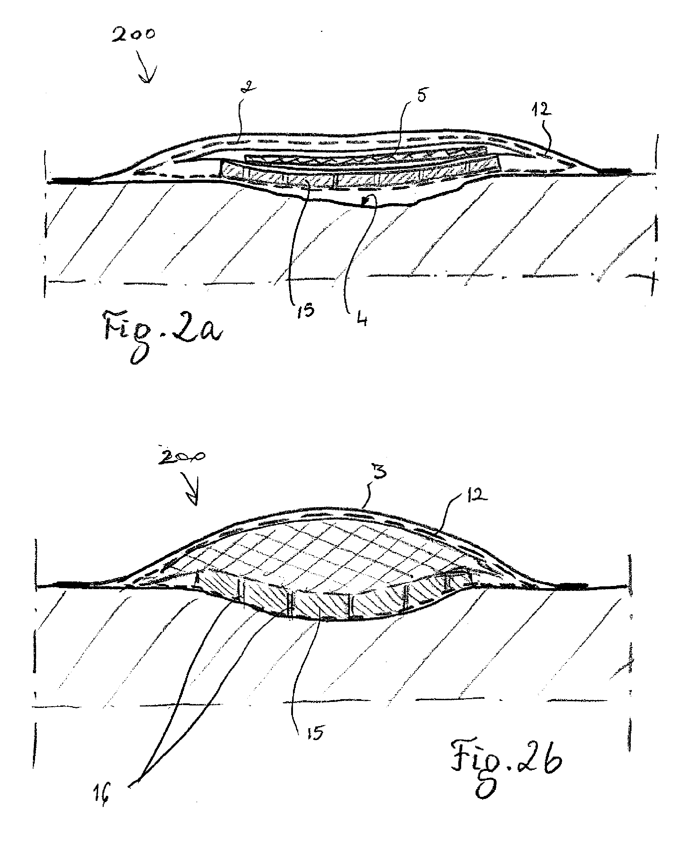

Die Figuren 2a und 2b zeigen einen anderen Wundverband 200, bestehend aus einem bei den Figuren 1a und 1b bereits beschriebenen Sachet 8, das mit einer gelochten schaumartigen Matte 15 unterlegt ist. Sowohl das Sachet 8, als auch die schaumartige Matte 15 ist von einer äußeren Umhüllung 12 umgeben, die aus demselben Hüllenmaterial besteht. Dementsprechend ist eine Konfiguration "Hülle in Hülle" gebildet, bei der die innere Hülle 2 (Sachet) von der äußeren Umhüllung 12 durch die Matte 15 beabstandet ist. Die schaumartige Matte 15 weist mehrere durchgehende Öffnungen 16 auf, die den Transport von flüssigen Wundexsudaten in Richtung Vliesmatte 5 beschleunigen und kanalisieren können. Nichtdestotrotz kann ein Teil von Wundexsudat von der schaumartigen Matte 15 übernommen werden, da diese offenporig ist und den ersten Kontakt mit dem Wundexsudat hat. Der übrige Funktionsmechanismus des Wundverbands 200 ist dem des Wundverbands 100 ähnlich. Das Abdeckungselement 3 ist ebenfalls elastisch und gibt ohne nennenswerte Gegenkräfte nach.

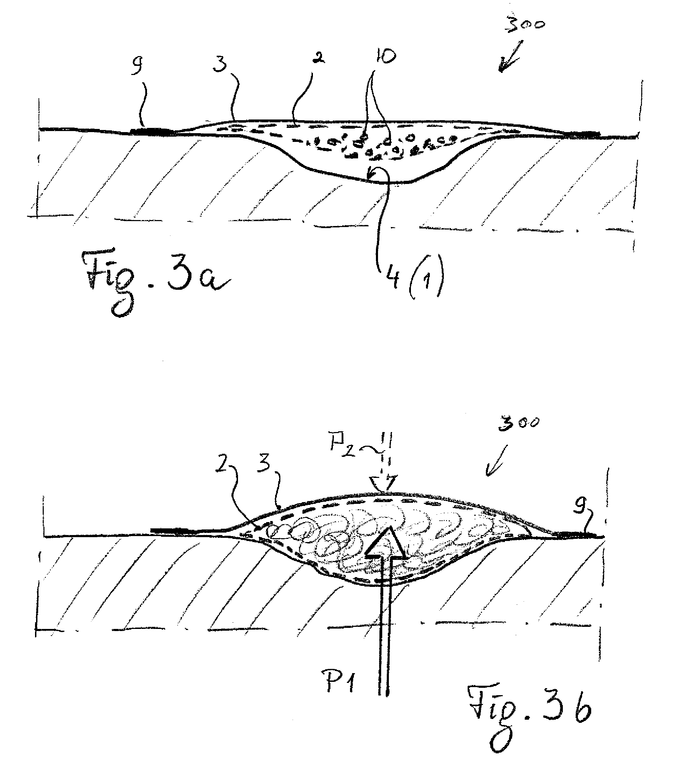

Ein weiterer, in den Figuren 3a und 3b dargestellter Wundverband 300 ist für tiefe, jedoch flächenmäßig kleinere Wunden gedacht. Innerhalb der schmiegsamen Hülle 2 befindet sich der Superabsorber 10 in Granulatform. Da die (steifere) Matte fehlt, legt sich die Hülle 2 mit dem Superabsorber einfacher an der tiefen Wunde 1 an. So übernimmt der Superabsorber allein die Funktion der Wundfüllung. Auch in dem Fall dehnt sich das Abdeckungselement 3 aus und gibt dem aufgequollenen Superabsorber 10 leicht nach. Das Abdeckungselement 3 ist aus natürlichem Seidengewebe (gammastrahl behandelte indische "Eri-Seide") gefertigt.

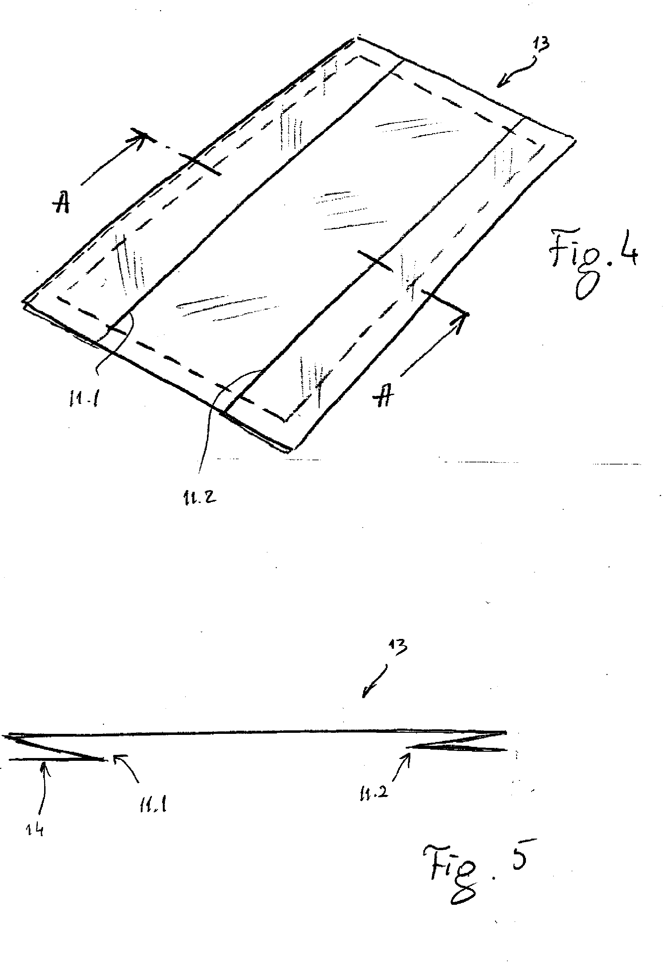

Die Figuren 4, 5 und 6 (Wundverband 400) zeigen, dass sich ein Abdeckungselement 13 bei Volumenzunahme der Matte frei entfalten kann, obwohl aus einer steiferen Kunststoffolie, in vorliegendem Fall aus Polyesterfolie, hergestellt ist. Zu diesem Zweck sind an dem in Fig. 4 gezeigten Folienabschnitt zwei sich gegenüber liegende (vgl. insbesondere Fig. 5) Falten 11.1, 11.2 eingearbeitet. Das Abdeckungselement 13 ist ebenfalls mit der umlaufenden, "unteren" Klebefläche 14 versehen. Für die Klarheit der Fig. 6 ist die Hülle 2 in ihrer ursprünglichen Lage, vor der Absorption gezeigt.

In einem weiteren, nicht dargestellten Ausführungsbeispiel verfügt das Abdeckungselement 13 über Falten an seinem ganzem Umfang.

### Bezugszeichenliste:

- 1: Wunde
- 2: Hülle
- 2.1,2.2: Hüllenwand
- 3: Abdeckungselement
- 4: Wundoberfläche
- 5: Matte
- 6: Wundkontur
- 7: Vlieskern
- 8: Sachet
- 9: Rand
- 10: Superabsorber
- 11.1,11.2: Falte
- 12: Hülle (äußere)
- 13: Abdeckungselement
- 14: Klebefläche
- 15: Matte
- 16: Öffnung
- 17.1, 17.2: Schicht
- P1; P2: Andrückkraft
- 100; 200: Wundverband
- 300; 400: Wundverband

## Patentansprüche

1. Wundverband (100; 200; 300; 400), insbesondere für stark exsudierende Wunden, aufweisend (i) eine Flüssigkeiten absorbierende Einheit (10) und (ii) ein folienartiges, an der gesunden Haut um die Wunde herum zu befestigendes Abdeckungselement (3; 13), wobei die Flüssigkeiten absorbierende Einheit bei Flüssigkeitsaufnahme an Volumen gewinnt und in diesem Zustand den jeweiligen Raum zwischen der Wundoberfläche und dem Abdeckungselement (3; 13) wenigstens teilweise ausfüllt,
**dadurch gekennzeichnet, dass**
- die Flüssigkeiten absorbierende Einheit durch das Abdeckungselement (3; 13) abgedeckt ist,
- das Abdeckungselement (3; 13) Mittel aufweist, die dazu beitragen, dass das Abdeckungselement in seinem auf der Haut des Patienten befestigten Zustand der Volumenzunahme der Flüssigkeiten absorbierenden Einheit nachgibt und sich kontinuerlich ausdehnt.

2. Wundverband gemäß Anspruch 1, **dadurch gekennzeichnet, dass** es sich bei der Flüssigkeiten absorbierenden Einheit um eine Einheit enthaltend superabsorbierende Substanz handelt, die zwischen der jeweiligen Wundoberfläche (4) und dem Abdeckungselement (3; 13) angeordnet ist

3. Wundverband nach Anspruch 1 oder 2, **dadurch gekennzeichnet, dass** das Abdeckungselement (3) aus einer streckbaren, plastisch und/oder elastisch verformbaren Folie, Gewebe oder Gewirke gefertigt ist.

4. Wundverband nach Anspruch 3, **dadurch gekennzeichnet, dass** das Abdeckungselement (3) aus einer Polymerfolie oder aus natürlichem Seidengewebe besteht.

5. Wundverband nach einem der vorherigen Ansprüche, **dadurch gekennzeichnet, dass** das Abdeckungselement (3) aus medizinischer Silikon- oder Latexfolie gefertigt ist.

6. Wundverband nach einem der vorherigen Ansprüche, **dadurch gekennzeichnet, dass** das Abdeckungselement (13) wenigstens teilweise gefaltet ist.

7. Wundverband nach einem der vorherigen Ansprüche, **dadurch gekennzeichnet, dass** die superabsorbierende Substanz (10) innerhalb einer flüssigkeitsdurchlässigen, aus zwei zueinander kongruenten Hüllenwänden (2.1, 2.2) bestehenden Hülle (2) angeordnet ist, die beim Auflegen auf die Wunde durch das Abdeckungselement (3) abgedeckt ist.

8. Wundverband einem der vorherigen Ansprüche, **dadurch gekennzeichnet, dass** die superabsorbierende Substanz (10) in oder an einer saugfähigen Matte (5) verteilt ist.

9. Wundverband nach einem der vorhergehenden Ansprüche, **dadurch gekennzeichnet, dass** die die superabsorbierende Substanz (10) umgebende Hülle (2) von wenigstens einer weiteren, ebenfalls saugfähigen Matte (15) unterlegt ist und dass sowohl die Hülle (2) als auch die Matte (15) innerhalb einer äußeren, flüssigkeitsdurchlässigen Umhüllung (12) angeordnet sind, die beim Auflegen auf die Wunde durch das Abdeckungselement (3) abgedeckt ist.

10. Wundverband nach Anspruch 8 oder 9, **dadurch gekennzeichnet, dass** die Matte (5; 15) aus Vliesfasern oder Schaumstoff besteht.

11. Wundverband nach Anspruch 8 oder 9, **dadurch gekennzeichnet, dass** die Matte (15) gelocht ist.

12. Wundverband nach Anspruch 10, **dadurch gekennzeichnet, dass** die aus Schaumstoff bestehende Matte (15) offenzellig oder geschlossenporig ist.

13. Wundverband nach einem der vorhergehenden Ansprüche, **dadurch gekennzeichnet, dass** das Abdeckungselement (3) flüssigkeitsundurchlässig und, optional, wasserdampfdurchlässig ist.

14. Wundverband nach einem der vorhergehenden Ansprüche, **dadurch gekennzeichnet, dass** das Abdeckungselement (3) mit Perforationen (8) versehen ist.

15. Wundverband nach einem der vorhergehenden Ansprüche, **dadurch gekennzeichnet, dass** die superabsorbierende Substanz (SAP), die Hülle und/oder die Umhüllung kleblos durch das Abdeckungselement (3; 13) abgedeckt ist.

16. Verwendung eines Abdeckungselementes wie stofflich in einem der Ansprüche 1 - 10 definiert zur Abdeckung einer Wundauflage.

17. Verwendung eines Abdeckungselementes gemäß Anspruch 16, wobei das Abdeckungselement an der gesunden Haut um die Wunde herum befestigt wird.

18. Verwendung eines Abdeckungselementes gemäß Anspruch 16, wobei die Wundauflage in aufgequollenem Zustand den jeweiligen Raum zwischen der Wundoberfläche und dem Abdeckungselement (3; 13) wenigstens teilweise ausfüllt.
